# EUROPÄISCHE PATENTSCHRIFT

(11) **EP 1 852 098 B1**
(45) Veröffentlichungstag und Bekanntmachung des Hinweises auf die Patenterteilung: **29.02.2012**
(21) Anmeldenummer: 07007068.5
(22) Anmeldetag: 04.04.2007
(51) Int. Cl.: A61F 13/20

(54) **Tampon mit verbesserter Haftung zwischen Hülle & Kern**
Pad for improved adhesion between core and wrapping
Tampon doté d'une adhérence améliorée entre l'enveloppe et le coeur

(30) Priorität: 02.05.2006 DE 102006020590
(43) Veröffentlichungstag der Anmeldung: 07.11.2007
(73) Patentinhaber: Sandler AG, 95126 Schwarzenbach/Saale (DE)
(72) Erfinder:

(56) Entgegenhaltungen:
- EP-A- 0 685 213
- WO-A-99/32061
- US-A- 4 543 098
- US-A1- 2005 113 786

## Beschreibung

Die vorliegende Erfindung betrifft einen Tampon (1) zur Aufnahme von Körperflüssigkeiten für die Damenhygiene, welcher eine verbesserte Lagenhaftung zwischen der Hüllschicht (2) und dem Kern (3) aufweist.

Für die Tamponumhüllung geeignete Abdeckmaterialien und für den Tamponkern geeignete Saugkörpermaterialien sind für Tampons der gattungsgemässen Art im Stand der Technik bekannt.

In EP 0401358 B1 ist ein Tampon dargestellt, der eine einschichtige Tamponumhüllung aus Fasern umfasst, die mit einem Pulverbindemittel thermisch miteinander verbunden und auf einen als Tamponkern fungierenden absorbierenden Wattebausch aufgepresst sind. Der Tamponkern besteht aus einem Gemisch aus Viskosefilamentfasern und Baumwolle.

Nachteilig dabei ist jedoch der aufwendige Herstellungsprozess, da hierbei eine zusätzliche, präzise Pulverdosierung sowie der kostenintensive Einsatz von Pulverbindemittel erforderlich ist. Eine zu gering eingebrachte Pulvermenge führt dazu, dass die Weichheit zu stark erhöht und die Festigkeit des Vlieses zu niedrig ist, während eine zu viel eingebrachte Pulvermenge einen zu steifen Warenausfall bewirkt, woraus letztendlich eine unzufriedenstellende Saugleistung des Tampons resultiert. Zudem besteht beim Produktionsprozess der Pulverbindung das Problem, dass das Pulver absolut homogen in den Faserverband eingebracht werden muss, d. h., ein ungenügendes bzw. nur partielles Verschmelzen des Pulverbindemittels und eine damit einhergehende zu schwache Verfestigung kann bei der Tamponherstellung mit hoher Verarbeitungsgeschwindigkeit zum Auftrennen oder Zerreissen der Tamponumhüllung führen.

EP 1194099 B1 offenbart ein Tampon mit einem perforierten Umhüllungsmaterial, welches mit einer absorbierenden Faserschicht wärmeverbunden ist. Das perforierte Umhüllungsmaterial kann u. a. Bikomponentenfasern umfassen, welche einen Polyester- oder Polypropylen-Kern und einen Polyethylen-Mantel aus HDPE, d. h., einem Polyethylen mit hoher Dichte, aufweisen.

Aufgrund der im Umhüllungsmaterial vorhandenen Perforierungen reduziert sich die Kontaktfläche zwischen dem Umhüllungsmaterial und dem Tamponkern, wodurch die Gefahr besteht, dass keine ausreichende Verbundhaftung erreicht wird, so dass es zu einer unerwünschten Delaminierung des Verbundes kommen kann.

EP 0397694 B1 zeigt Tampons mit einer flüssigkeitsdurchlässigen Abdeckschicht, die einen aus hydrophilen Cellulose-Fasern bestehenden, saugfähigen Kern von zylindrischer Form umgibt. Die Abdeckschicht ist entweder mittels einer Klebverbindung oder vorzugsweise mittels Heißsiegeln an der Oberfläche des saugfähigen Kerns befestigt. Die Abdeckschicht umfasst ein Verbundmaterial aus einem thermoplastischen Polymer mit höherem Schmelzpunkt und einem thermoplastischen Polymer mit niedrigerem Schmelzpunkt. Die heißsiegelbare Schicht soll ein Verschieben der Abdeckschicht beim Einführen oder Herausnehmen des Tampons verhindern. Allerdings kommt es spätestens nach einer gewissen Tragezeit des Tampons zu einer rapiden Abnahme der Haftkraft zwischen der aus synthetischen Polymeren bestehenden Abdeckschicht und dem aus cellulosischen Fasern zusammengesetzten saugfähigen Kern, da der Kern aufgrund der Feuchtigkeitsaufnahme sehr stark aufquillt und überdies deformiert wird und somit erhöhte Spannungskräfte an der Verbindungsfläche zwischen Kern und Abdeckschicht auftreten. Infolge dessen besteht bei Tampons aus dieser Lehre immer noch die Gefahr des Verschiebens der Abdeckschicht bzw. das Zurückbleiben des Kerns im Vaginalbereich beim Herausnehmen des gebrauchten Tampons.

DE 19753665 C2 handelt von einem Tampon für die Frauenhygiene oder medizinische Zwecke. Als Abdeckmaterial dient ein thermisch verfestigter und oberflächengeglätteter Vliesstoff aus Bikomponenten-Stapelfasern wie z. B. PE/PET Mantel/Kern-Art. Der Tamponrohling für den Tamponkern besteht aus einem kardierten, hydrophilen Faservlies und ist mit dem Abdeckmaterial mittels Heißsiegeln verbunden. Die mittels Kalandrieren erzielte Oberflächenglättung des Abdeckmaterials führt zu einer Verringerung der Reibung an der äußeren Oberfläche des Tampons und soll dazu beitragen, dass der Tampon leichter in eine Körperhöhle eingeführt und ebenso wieder entfernt werden kann. Die Erfinder haben dabei jedoch nicht erkannt, dass für die erforderliche Gewährleistung des Tragekomforts während der gesamten Tragezeit und zur Vermeidung der Delamination beim Entfernen des Tampons eine Verbesserung der Haftkraft zwischen Tamponkern und Abdeckvliesstoff von absoluter Relevanz ist. Somit besteht auch bei derartigen Tampons nach wie vor die Gefahr, dass sich der Abdeckvliesstoff beim Entnehmen des Tampons aus der Körperhöhle ablöst und darin verbleibt.

EP 1189566 B1 offenbart ein Verfahren bzw. eine Vorrichtung zum Verbinden eines thermoplastischen Abdeckmaterials mit einem Faservlies, welches bei der Herstellung eines Tampons Anwendung finden kann. Hierbei wird das Abdeckmaterial, das vorzugsweise Polyethylen enthält, auf eine Faserbahn, die Baumwolle und Rayon enthält, aufgelegt und nach Passage eines von einer Siegel- und Riffelwalze gebildeten Walzenspalts unter Wärmeeinwirkung miteinander verbunden. Anschließend erfolgt mit Hilfe eines auf der Siegelwalze befindlichen Bügelelements die Kalandrierung der Faserbahn unter Wärmeeinwirkung. Für die Kalandrierung ist der Temperaturbereich niedriger als bei der Versiegelung, d. h., geringer als der Erweichungspunkt des thermoplastischen Materials im Abdeckmaterial. Die hier beschriebene Lehre zielt auf eine Vorrichtung und ein Verfahren zum Verbinden von Tamponumhüllung und Tamponkern sowie zum Glätten der äußeren Oberfläche der Tamponumhüllung ab. Analog zur vorstehenden Lehre kann mit der hier dargestellten Vorrichtung und dem hier geschilderten Verfahren kein Tampon geschaffen werden, der eine optimale Haftung zwischen Tamponumhüllung und Tamponkern aufweist.

US 4,543,098 offenbart einen Tampon mit dehnfähiger Komponente und Mikrofaser-Einlage. Die äußere Lage der Transferschicht enthält gekräuselte thermoplastische Fasern, während die innere Lage neben thermoplastischen zusätzlich zellulosische Fasern enthalten kann. Die Verbindung der Lagen erfolgt entweder mittels Thermobonding-Kalander oder mittels Ultraschallverschweißungs-Vorrichtung.

In US 2005/0113786 A1 wird ein Tampon aus komprimiertem faserigen Material für Damenhygiene-Anwendungen beschrieben, dessen Außenfläche eine Vielzahl von ausgesparten Abschnitten aufweist, wodurch dem Träger eine erhöhte Aufnahmefähigkeit der Menstruationsflüssigkeit sowie eine ästhetisch ansprechende äußere Optik verschafft werden soll. Wegen der vorhandenen Aussparungen verringert sich die Kontaktfläche zwischen Umhüllungsmaterial und Tamponkern zwangsläufig, so dass die Gefahr besteht, dass nach erfolgter Flüssigkeitsaufnahme und dadurch geschehener Ausdehnung und Verlust der Form des Tampons die Verbundhaftung zwischen Umhüllungsmaterial und Tamponkern bei Einwirkung von mechanischer Einwirkung nicht mehr ausreichend ist, um eine Delaminierung zu verhindern.

WO 99/32061 A1 handelt von einem Tampon mit einer mehrschichtigen Hülle und einem absorbierenden Kern. Abgesehen von einer Verbesserung des Tragekomforts soll ein nach dieser Lehre gefertigter Tampon zusätzlich den Einführ- und Herausnahmekomfort erhöhen, indem die Außenschicht der mehrschichtigen Hülle mit einer glatten Oberfläche versehen ist. Eine Optimierung der Haftung zwischen der Umhüllung und dem Kern wird bei dieser Erfindung nicht berücksichtigt. Ein nach dieser Erfindung produzierter Tampon kann deshalb auch keine optimale Haftung zwischen Umhüllung und Kern besitzen.

Den Materialien des Standes der Technik ist allen gemeinsam, dass die Haftung zwischen der Hüllschicht (2) und dem Kern (3) gegen Ende der Gebrauchszeit, bedingt durch die Quellung und damit dem Verlust der ursprünglichen Form des Tampons, drastisch abfällt.

Der Verlust dieser Haftung kann beispielsweise bei einem Menstrualtampon schlimmstenfalls dazu führen, dass sich die Hüllschicht (2) beim Entnehmen des Tampons (1) aus der Körperhöhle ablöst und in der Körperhöhle verbleibt. Es gilt, derartig erhebliche Gesundheitsrisiken für die Trägerin im Voraus vollkommen auszuschließen.

Nach dem Stand der Technik wird die Hüllschicht (2) mit dem Kern (3) mittels eines thermischen Prozesses miteinander verpresst und verschweißt. Geeignete Verfahren dafür sind neben in EP 1189566 B1 auch beispielsweise in DE 19753665 C2 offenbart. Die Haftung zwischen der Hüllschicht (2) und dem Kern (3) wird dabei nur durch die schmelzbare Komponente in der Hüllschicht (2) gewährleistet. Der Kern (3) besteht dabei zu 100% aus Viskosefasern.

Ist dieser Tampon nun im Gebrauch, beginnt die Viskosefaser und damit der Kern (3) sich mit Flüssigkeit voll zusaugen und quillt. Dabei geht die ursprüngliche Form verloren und der Tampon (1) nimmt an Umfang zu.

Einhergehend mit dieser strukturellen Veränderung des Tampons (1) ist aber auch die Abnahme der Haftung zwischen der Hüllschicht (2) und dem Kern (3). Die durch das Verpressen zwischen der Hülle (2) und dem Kern (3) entstandenen Verbindungsstellen an der Lagen-Grenzschicht (4) platzen zum einen durch die veränderte Topographie der Tamponoberfläche und zum anderen durch die Quellung der die den Kern (3) bildenden Fasern auf.

In der Folge nimmt die Haftung zwischen Kern (3) und Hüllschicht (2) ständig weiter ab.

Aufgabe der Erfindung war es daher, diesen Nachteil des Standes der Technik zu vermeiden und einen Tampon bereitzustellen, der eine verbesserte Haftung zwischen Hülle (2) und Kern (3) aufweist.

Diese Aufgabe wird gelöst durch das erfindungsgemässe Einbringen von schmelzbaren, thermoplastischen Fasern (6) sowohl in die Hüllschicht (2) als auch in den Kern (3). Somit werden neben den bereits Eingangs erwähnten Bindungsstellen zusätzliche, rein zwischen den Fasern (6) existierende Bindungsstellen aufgebaut.

Da die Fasern (6) nicht auf Basis von saugfähigen und daher quellenden Polymeren sind, bleiben die Bindungsstellen zwischen den Fasern in der Hüllschicht (2) und dem Kern (3) auch im gequollenen Zustand bestehen und verbessern daher erfindungsgemäß die Haftung der Hüllschicht (2) auf dem Kern (3).

Der erfindungsgemäße Tampon (1) kann durch die Maßnahmen, welche in den Unteransprüchen 2 bis 5 genannt sind, weitergehend sinnvoll ausgestaltet werden.

Es hat sich als vorteilhaft herausgestellt, wenn die Hüllschicht (2) aus einem mehrlagigen kardierten Stapelfaservlies besteht, deren Oberseite (2a) zu 100 % aus Matrixfasern (5) besteht und deren Unterseite (2b) aus einer intimen Mischung von Matrixfasern (5) und schmelzbaren, thermoplastischen Fasern (6) besteht.

Zudem hat es sich als günstig erwiesen, wenn der Kern (3) aus einem mehrlagigen kardierten Stapelfaservliesstoff besteht, dessen Oberseite (3a) aus einer intimen Mischung von schmelzbaren, thermoplastischen Fasern (6) und Saugfasern (7) besteht und dessen Unterseite (3b) zu 100 % aus Saugfasern (7) besteht.

Ebenfalls als vorteilhaft hat sich herausgestellt, dass die Matrixfasern (5) der Hüllschicht (2) aus Mono- und/oder Bikomponentenfasern auf Basis von Polyolefinen bestehen.

Eine weitere vorteilhafte Ausgestaltung - wie in Anspruch 2 angeführt - der vorliegenden Erfindung wurde gefunden, indem die Matrixfasern (5) der Hüllschicht (2) aus Bikomponentenfasern auf Basis von Polyolefinen und Polyestern bestehen.

Insbesondere ist es von zusätzlichem Vorteil, wenn - wie in Anspruch 3 angeführt - die schmelzbaren, thermoplastischen Fasern (6) in der Hüllschicht (2) und die im Kern (3) synthetischen, bikomponenten Fasern auf Basis von Polyolefinen und Polyestern sind.

Als weitere vorteilhafte Variante der vorliegenden Erfindung kommt in Frage, dass die schmelzbaren, thermoplastischen Fasern (6) in der Hüllschicht (2) und im Kern (3) synthetische, bikomponente Fasern auf Basis von Polyolefinen und Polyestern sind.

Ferner ist es sinnvoll, dass die schmelzbaren, thermoplastischen Fasern (6) in der Hüllschicht (2) und im Kern (3) synthetische, mono- und oder bikomponente Fasern sind, auf Basis von Polyolefinen, welche ein Polymer enthalten, welches eine verbesserte Haftung zu zellulosischen Materialien aufbaut.

Überdies ist für das optimale Erreichen der Absorptionsfunktion vorteilhaft, dass die Saugfasern (7) auf zellulosischer Basis sind - wie in Anspruch 5 angeführt. Ein weitere vorteilhafte Ausgestaltung des erfindungsgemässen Tampons (1) besteht darin, dass der Anteil der schmelzbaren, thermoplastischen Fasern (6) in der intimen Mischung der Hüllschicht (2) zwischen 5 und 50 Gewichtsprozent bezogen auf das Gesamtgewicht der Hüllschicht (2) beträgt.

Einen zusätzlichen Vorteil bringt es, wenn der Anteil der schmelzbaren, thermoplastischen Fasern (6) in der intimen Mischung des Kerns (3) zwischen 2,5 und 50 Gewichtsprozent bezogen auf das Gesamtgewicht des Kerns (3) beträgt.

Weitere Vorteile der vorliegenden Erfindung gehen im Einzelnen aus der nachstehenden Beschreibung hervor.

Der erfindungsgemäße Tampon (1) wird dabei in einem ersten Beispiel wie folgt hergestellt.

Zunächst wird die Hüllschicht (2) aus einem kardierten Stapelfaservliesstoff - wie in dem Fachbuch "Vliesstoffe", S. 145 ff, herausgegeben von Abrecht, Fuchs und Kittelmann, erschienen im WILEY-VCH-Verlag, beschrieben - aus einer Mischung von Matrixfasern (5) und schmelzbaren, thermoplastischen Fasern (6) erzeugt. Praxisüblich werden dabei Mischungen aus Polypropylenfasern als Matrixfaser (5) und Polypropylen / Polyethylen Kern-/ Mantelfasem als thermoplastische Fasern (6) eingesetzt.

Die Fasern sind dabei in homogener Verteilung miteinander gemischt und weisen Mischungsverhältnisse von 5 bis 90 Gewichtsprozent Matrixfasern (5) zu 95 bis 10 Gewichtsprozenten thermoplastische Fasern (6) auf.

Der flüssigkeitsabsorbierende Kern (3) umfasst im Unterschied zum Stand der Technik erfindungsgemäß ein kardiertes Vlies aus einer Mischung von Saugfasern (7) und thermoplastischen Fasern (6). Diese Fasern sind ebenfalls in homogener Verteilung miteinander gemischt und weisen Mischungsverhältnisse von 5 bis 20 Gew.% thermoplastischer Fasern (6) und 95 bis 80 Gew-% Matrixfasern (5) auf.

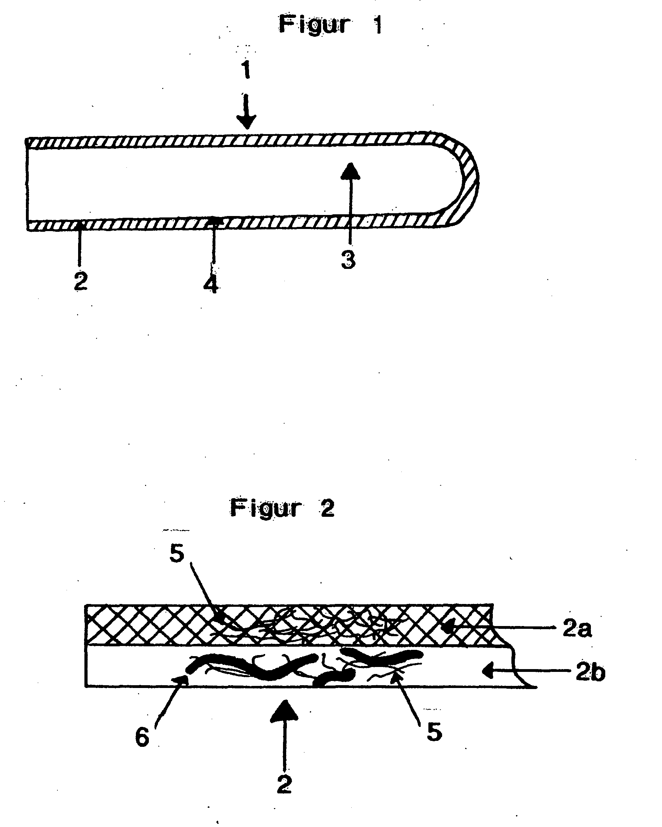

In den Figuren 1 bis 4 sind vorteilhafte Ausgestaltungen der Erfindung dargestellt.

Figur 1 zeigt den schematischen Aufbau des erfindungsgemässen Tampons (1). Der aus einem solch gearteten kardierten Vlies zylinderförmig gerollte und definiert komprimierte Kern (3) ist mit der Hüllschicht (2) unter Aktivierung der thermoplastischen Fasern unter Anwendung von Druck und Wärme an seiner Oberfläche verbunden.

Zur Verdeutlichung der mit erfindungsgemäß hergestellten Tampons erzielbaren Lagenhaftung wurden Messungen an diversen Tamponmaterialien vorgenommen, indem die zwischen der Hüllschicht (2) und dem Kern (3) herrschende Trennfestigkeit mit einer universalen Zugfestigkeitsprüfmaschine an streifenförmigen Prüfkörpern - ergo an aufgerollten Tampons - ermittelt wurde. Die dazugehörigen Ergebnisse sind in der Tabelle 2 dargestellt.

Ergänzend hierzu wird in der Tabelle 1 angegeben, aus welchen Materialzusammensetzungen jeweils die Hüllschicht (2) und der Kern (3) der getesteten Tamponmaterialien bestehen.

An diversen nach dem Stand der Technik hergestellten Tampons und am erfindungsgemäss hergestellten Tampon wurde die Haftung der Hüllschicht (2) zum Kern (3) hin untersucht. Die Lagenhaftung wurde dabei am nach dem Verpressvorgang wieder aufgerollten Tampon in Längs- (Roll-) Richtung (MD) und auch quer zur Rollrichtung (CD) mit einer industrieüblichen Universalprüfmaschine bestimmt, d. h. die Tests wurden an streifenförmigen Material-Prüfkörpern durchgeführt. Dabei wurde die Hülle (2) in der oberen Klemme eingespannt, der Saugkern (3) in die untere. Die Abzugsgeschwindigkeit betrug 100 mm/min.

Wie der Tabelle 2 entnommen werden kann, weist das erfindungsgemäße Material bei der Ermittlung der Lagenhaftung deutlich höhere Minimalkräfte auf als das beispielhaft angeführte Material nach dem Stand der Technik.

Die Gefahr, dass sich die Hüllschicht (2) vom Saugkern (3) ablöst, ist damit bei Verwendung erfindungsgemäß hergestellter Materialien deutlich reduziert.

Eine weitere Verbesserung der Lagenhaftung lässt sich in einer nachstehend bevorzugten Ausführungsform erzielen.

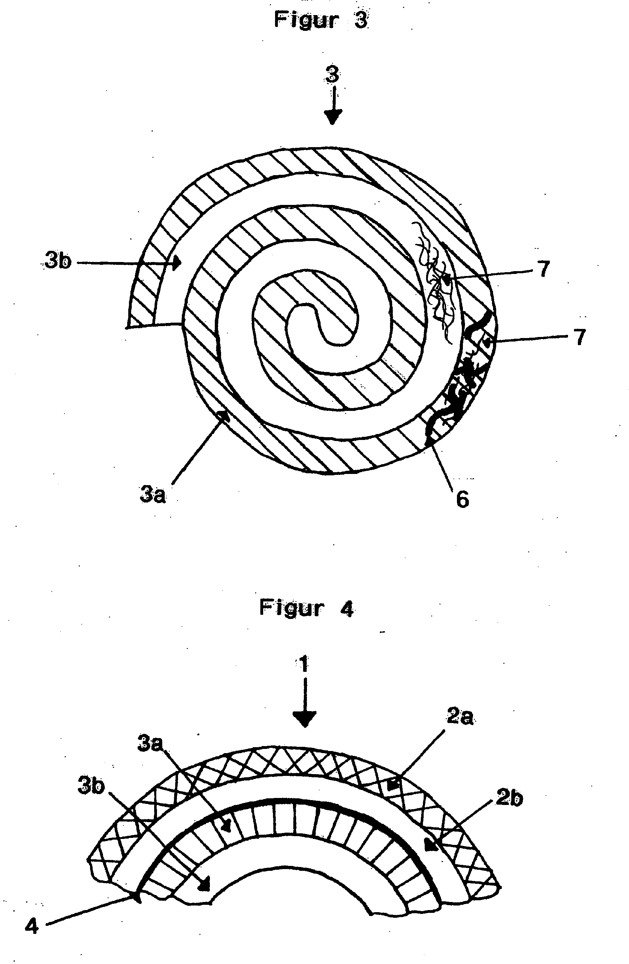

Dabei besteht der in Figur 3 im Querschnitt dargestellte Kern (3) aus einem zweilagigen Stapelfaservlies, dessen Oberseite (3a) eine intime Mischung aus 5-20% thermoplastischer Fasern (6) mit 95-80% Saugfasern (7) darstellt und dessen Unterseite (3b) zu 100% aus Saugfasern (7) besteht.

Analog dazu ist auch die in Figur 2 im Querschnitt dargestellte Hüllschicht (2) als zweilagiges Stapelfaservlies ausgebildet. Die Oberseite (2a) besteht dann zu 100% aus Matrixfasern (5), die Unterseite (2b) aus einer intimen Mischung von 0-80 % Matrixfasern (5) und 20-100% thermoplastischen Fasern (6).

Beim Rollen des Tampons ist dann so zu verfahren, dass die Oberseite (3a) des Kerns (3) zur Unterseite (2b) der Hüllschicht (2) hin zeigt.

Durch die speziell gewählte Anordnung der Ober- und Unterseiten von Hüllschicht (2) und Kern (3) - wie aus Figur 4 ersichtlich - kommen an der Lagen-Grenzschicht (4) die Materialschichten mit erhöhter Bindungsaffinität aufeinander zu liegen.

Dadurch ergibt sich eine vergrößerte Anzahl von möglichen Bindungsstellen, die wiederum die Lagenhaftung zwischen Hülle (2) und Kern (3) positiv beeinflussen.

Figur 3 zeigt in einer Querschnittsdarstellung eine bevorzugte Ausgestaltung des gerollten Kerns (3) des erfindungsgemässen Tampons (1).

Hierbei besteht der Kern aus einem zweilagigen kardierten Stapelfaservliesstoff, dessen Oberseite (3a) eine intime Mischung von schmelzbaren, thermoplastischen Fasern (6) und Saugfasern (7) umfasst, und dessen Unterseite (3b) zu 100 % aus Saugfasern (7) gebildet ist.

Eine weitere Verbesserung kann erreicht werden, wenn als Ausgangspolymere für die thermoplastischen Fasern (6) Polymere eingesetzt werden, die durch eine chemische Modifikation eine verbesserte Haftung zu zellulosischen Fasern aufbauen. Das Grundprinzip dazu ist in DE 29913054 U1 beschrieben.

Durch die vorgenannten Maßnahmen ist es letztendlich möglich geworden, die Gefahr einer Delaminierung der Hülle (2) vom Kern (3) entscheidend zu minimieren.

**Tabelle 1: Herstellung der Vergleichsmuster**

| Parameter | Einheit | Tampon nach dem Stand der Technik | **Tampon nach dem Stand der Technik Vergleichsbeispiel 1** | Erfindungsgemäßer Tampon |
|---|---|---|---|---|
| Zusammensetzung der Hüllschicht (2) | | 100% Bikomponenten -faser PP-Kern / PE-Mantel | 100% Bikomponentenfaser PP-Kern / PE-Mantel | 2-Lagenvlies, Oberseite (2a): 100% Matrixfaser (5) (PP-Stapelfaser) , Unterseite (2b): Intime Mischung aus 50 % Matrixfaser (5) (PP-Stapelfaser) und 50 % thermoplastische Faser (6) aus PP/PE-Stapelfaser. |
| Zusammensetzung des Kerns (3) | | 100% Zellulosefaser | 85% Zellulosefaser 15% Bikomponentenfaser PP-Kern / PE-Mantel | 2-Lagenvlies, Oberseite (3a): intime Mischung aus 85% Zellulosefaser 15% thermoplastische Faser (6) PP / PE Stapelfasern. Unterseite (3b): 100% Zellulosefaser |
| Temperatur beim Verpressen | Grad C | 144 | 144 | 144 |
| Zeit für das Verpressen | Sec | 5 | 5 | 5 |

**Tabelle 2: Ergebnisse der Vergleichsmuster**

| Parameter | Einheit | Tampon nach dem Stand der Technik | **Tampon nach dem Stand der Technik Vergleichsbeispiel 1** | Erfindungsgemäßer Tampon |
|---|---|---|---|---|
| Mittlere Lagenhaftung zwischen Hülle (2) und Kern (3) in MD-Richtung | N/5cm | 0,70 | 1,00 | 1,24 |
| Mittlere Lagenhaftung zwischen Hülle (2) und Kern (3) in CD-Richtung | N/5cm | 0,61 | 0,95 | 1,15 |
| Minimale Lagenhaftung zwischen Hülle (2) und Kern (3) in MD-Richtung | N/5cm | 0,30 | 0,64 | 0,74 |
| Minimale Lagenhaftung zwischen Hülle (2) und Kern (3) in CD-Richtung | N/5cm | 0,10 | 0,44 | 0,52 |

### Bezugszeichen-Liste

1 = Tampon
2 = Hüllschicht
2a = Oberseite Hüllschicht
2b = Unterseite Hüllschicht
3 = Kern
3a = Oberseite Kern
3b = Unterseite Kern
4 = Lagen-Grenzschicht
5 = Matrix-Faser
6 = thermoplastische Faser
7 = Saug-Faser

## Patentansprüche

1. Tampon (1) zur Aufnahme von Körperflüssigkeiten mit verbesserter Lagenhaftung zwischen Hüllschicht (2) und Kern (3), wobei die Hüllschicht (2) und der Kern (3) aus kardiertem Stapelfaservlies bestehen und wobei die Hüllschicht (2) neben einer Matrixfaser (5) eine schmelzbare, thermoplastische Faser (6) und der Kern (3) neben einer Saugfaser (7) eine schmelzbare thermoplastische Faser (6) enthalten, wobei beim Rollen des Tampons so verfahren wird, dass die Oberseite (3a) des Kerns (3) zur Unterseite (2b) der Hüllschicht (2) hin zeigt,
**dadurch gekennzeichnet, dass**
die Oberseite (2a) der mehrlagigen und kardierten Hüllschicht (2) zu 100 % aus Matrix-Fasern (5) und deren Unterseite (2b) aus einer intimen Mischung von Matrix-Fasern (5) und schmelzbaren thermoplastischen Fasern (6) bestehen und, dass die Oberseite (3a) des mehrlagigen und kardierten Kerns (3) aus einer intimen Mischung von schmelzbaren thermoplastischen Fasern (6) mit Saugfasern (7) und dessen Unterseite (3b) zu 100 % aus Saugfasern (7) besteht.

2. Tampon nach Anspruch 1,
**dadurch gekennzeichnet, dass**
die Matrixfasern (5) der Hüllschicht (2) aus Bikomponentenfasern auf Basis von Polyolefinen und Polyestern bestehen.

3. Tampon nach einem der Ansprüche 1-2,
**dadurch gekennzeichnet, dass**
die schmelzbaren, thermoplastischen Fasern (6) in der Hüllschicht (2) und im Kern (3) synthetische, bikomponente Fasern auf Basis von Polyolefinen und Polyestern sind.

4. Tampon nach einem der Ansprüche 1-2,
**dadurch gekennzeichnet, dass**
die schmelzbaren, thermoplastischen Fasern (6) in der Hüllschicht (2) und im Kern (3) synthetische, mono- und/oder bikomponente Fasern auf Basis von Polyolefinen sind.

5. Tampon nach einem der Ansprüche 1-2,
**dadurch gekennzeichnet, dass**
die Saugfasern (7) auf zellulosischer Basis sind.

## Claims

1. A tampon (1) for absorbing body fluids, with improved layer adhesion between covering layer (2) and core (3), in which the covering layer (2) and the core (3) consist of carded staple fibre fleece and in which the covering layer (2) besides a matrix fibre (5) contains a melt-able, thermoplastic fibre (6), and the core (3), besides an absorbent fibre (7), contains a melt-able thermoplastic fibre (6), in which, upon rolling the tampons, one proceeds in a manner that the top felt side (3a) of the cores (3) points towards the bottom side (2b) of the covering layer (2),
**characterised in that**
the top felt side (2a) consists of the multiple layer and carded covering layer (2) made of 100% matrix fibre (5) and their bottom side (2b) consists of an intimate mixture of matrix fibres (5) and melt-able thermoplastic fibres (6) and, that the top felt side (3a) of the multiple layers and carded cores (3) are made of an intimate mixture of melt-able thermoplastic fibres (6) with absorbent fibres (7) and its bottom side (3b) consists of 100 % of absorbent fibres (7).

2. The tampon according to claim 1,
**characterised in that**
the matrix fibres (5) of the covering layer (2) consist of bi-component fibres based on polyolefin and polyester.

3. The tampon according to one of claims 1-2,
**characterised in that**
the melt-able, thermoplastic fibres (6) in the covering layer (2) and in the core (3) are synthetic, bi-component fibres based on polyolefin and polyester.

4. The tampon according to one of claims 1-2,
**characterised in that**
the melt-able, thermoplastic fibres (6) in the covering layer (2) and in the core (3) are synthetic, bi-component fibres based on polyolefin and polyester.

5. The tampon according to one of claims 1-2,
**characterised in that**
the absorbent fibres (7) are of cellulose basis.

## Revendications

1. Tampon (1) destiné à absorber des liquides corporels et offrant une meilleure adhérence des couches entre la couche enveloppante (2) et l'âme (3), sachant que la couche enveloppante (2) et l'âme (3) se composent de non-tissé cardé de fibres discontinues et sachant que la couche enveloppante (2) contient, outre une fibre matricielle (5), une fibre (6) fusible thermoplastique, et que l'âme (3) contient, outre une fibre absorbante (7), une fibre (6) fusible thermoplastique, sachant qu'au moment de rouler le tampon on procède de sorte que le dessus (3a) de l'âme (3) regarde le dessous (2b) de la couche enveloppante (2),
**caractérisé en ce que**
le dessus (2a) de la couche enveloppante (2) multicouche et cardée se compose à 100 % de fibres matricielles (5) et le dessous (2b) d'un mélange intime de fibres matricielles (5) et de fibres (6) fusibles thermoplastiques, et **en ce que** le dessus (3a) de l'âme (3) multicouche et cardée se compose d'un mélange intime de fibres (6) fusibles thermoplastiques et de fibres absorbantes (7), et le dessous (3b) de l'âme se compose à 100 % de fibres absorbantes (7).

2. Tampon selon revendication 1,
**caractérisé en ce que**
les fibres matricielles (5) de la couche enveloppante (2) se composent de fibres bicomposants à base de polyoléfines et de polyesters.

3. Tampon selon l'une des revendications 1-2,
**caractérisé en ce que**
les fibres (6) fusibles thermoplastiques présentes dans la couche enveloppante (2) et dans l'âme (3) sont des fibres synthétiques bicomposants à base de polyoléfines et de polyesters.

4. Tampon selon l'une des revendications 1-2,
**caractérisé en ce que**
les fibres (6) fusibles thermoplastiques présentes dans la couche enveloppante (2) et dans l'âme (3) sont des fibres synthétiques mono et bicomposants à base de polyoléfines.

5. Tampon selon l'une des revendications 1 - 2,
**caractérisé en ce que**
les fibres absorbantes (7) sont à base de cellulose.
